# EUROPEAN PATENT APPLICATION

(11) **EP 4 026 541 A1**
(43) Date of publication of application: **13.07.2022**
(21) Application number: 22150559.7
(22) Date of filing: 07.01.2022
(51) Int. Cl.: A61K 9/20, A61K 31/4545

(54) **A FILM COATED TABLET OF APIXABAN**

(30) Priority: 08.01.2021 TR 202100250; 15.02.2021 TR 202102085
(71) Applicant: SANOVEL ILAC SANAYI VE TICARET A.S., Istanbul 34460 (TR)
(72) Inventor: TASKIN, Abdullah, Istanbul (TR); MARASLI, Mustafa, Istanbul (TR); PALANTOKEN, Arzu, Istanbul (TR); SUNEL, Fatih, Istanbul (TR)
(74) Representative: Sevinç, Erkan

(57) **Abstract**

The present invention relates to a film coated tablet comprising apixaban and at least one binder, wherein at least one binder is dissolved in solvent comprising water. Furthermore, the present invention discloses a process for the preparation of a film coated tablet formulation comprising apixaban. The process is simple, rapid, cost effective, time-saving and industrially convenient process and is created to eliminate the disadvantages of active ingredient.

## Description

### Field of the Invention

The present invention relates to a film coated tablet comprising apixaban and at least one binder, wherein at least one binder is dissolved in solvent comprising water. Furthermore, the present invention discloses a process for the preparation of a film coated tablet formulation comprising apixaban. The process is simple, rapid, cost effective, time-saving and industrially convenient process and is created to eliminate the disadvantages of active ingredient.

### Background of the Invention

Apixaban is a highly selective, orally bioavailable, and reversible direct inhibitor of free and clot-bound factor Xa. Factor Xa catalyzes the conversion of prothrombin to thrombin, the final enzyme in the coagulation cascade that is responsible for fibrin clot formation. Apixaban has no direct effect on platelet aggregation, but by inhibiting factor Xa, it indirectly decreases clot formation induced by thrombin.

The chemical name of apixaban is 1-(4-methoxyphenyl)-7-oxo-6-[4-(2-oxopiperidin-1-yl)phenyl]-4,5,6,7-tetrahydro-1H-pyrazolo[3,4-c]pyridine-3-carboxamide and its chemical structure is shown in the Formula 1.

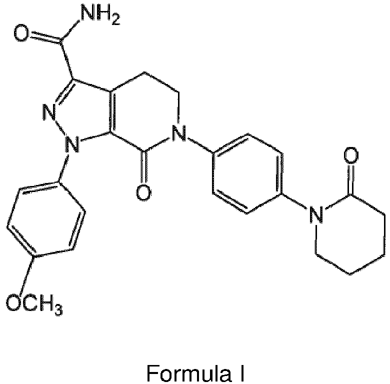

Apixaban molecule is disclosed in U.S. Patent No. 6,967,208.

Apixaban, sold under the tradename Eliquis, is an anticoagulant for the treatment of venous thromboembolic events. It is taken orally. It is a direct factor Xa inhibitor and apixaban has poor solubility in water (0.028 mg/mL at 24 °C) and a relatively low oral bioavailability (about 50% for a single 10 mg dose).

Apixaban has poor solubility in water (0.028 mg/mL at 24 °C) and a relatively low oral bioavailability (about 50% for a single 10 mg dose).

In prior art, there are many patents which disclose oral pharmaceutical dosage forms comprising apixaban.

Pharmaceutical compositions comprising the poorly soluble active ingredient apixaban are known in the art. In particular, several publications teach preparing pharmaceutical compositions comprising apixaban having a particular particle size, for example, WO 2017/182908 A1 describes compositions prepared by using apixaban of particles d(0.9) about 20 µm.

US 2013/0045245 application discloses a composition with improved dissolution rate by using crystalline apixaban having D90 equal to or less than 89 µm and a method for improving the dissolution rate of apixaban as a water insoluble drug by controlling the particle size distribution.

For the low solubility of apixaban, we have seen work done with many particle sizes of apixaban in the prior art. However, the above described methods for improving the dissolution rate by reducing particle size has a problem that the drug distribution in vivo varies depending on the particle size variation.

There is still a need in the art to provide an improved a tablet of apixaban that exhibits improved dissolution rate and good content uniformity, as a property optionally and preferably independent from a particle size of apixaban that may be used.

The present invention discloses a film coated tablet comprising at least one binder which is dissolved in solvent comprising water and apixaban. So, this formulation overcomes the above problems. In this way, an even higher quality of the formulations can be guaranteed with independent of the physical and chemical parameters of apixaban.

### Detailed Description of the Invention

The main object of the present invention is to eliminate problems and bringing additional advantages to the relevant prior art.

The more clearly main object of the present invention is to provides a film coated tablet comprising apixaban having desired dissolution profile and desired stability with independent of the physical and chemical parameters of apixaban.

Another object of the present invention is to provide a tablet having improved content uniformity.

Another object of the present invention is to provides a process for a film coated tablet comprising apixaban. The process is a simple, rapid, cost effective, time-saving and industrially convenient method.

The term "apixaban" as used herein refers to apixaban in the form of free base or in the form of pharmaceutically acceptable salts, crystalline polymorph, solvates, hydrates, esters or in an amorphous form or mixtures thereof.

According to one embodiment of the present invention, a film coated tablet comprises apixaban and at least one binder, wherein at least one binder is dissolved in solvent comprising water.

It has surprisingly been found that a film coated tablet of excellent content uniformity, good flowability and showing improved dissolution can be obtained when at least one binder and a solvent comprising water is formulated together and then adding apixaban into solvent. Also, good solubility is achieved independent of the particle size of the apixaban.

In one embodiment, the weight ratio of at least one binder to apixaban is between 1:1 and 5:1, preferably between 1:1 and 3:1. It has been observed that, this ratio is an important factor for achieving desired dissolution profile. When apixaban and at least one binder are used in these ratios, it is also easier to provide a formulation has improved content uniformity with wet granulation.

According to one embodiment of the present invention, the amount of apixaban is between 1.0% and 10.0% by weight in the total formulation. Preferably, it is between 1.0% and 8.0% or between 1.0% and 5.0% or between 1.0% and 3.0% by weight in the total formulation.

Suitable solvent is selected from the group comprising water, dichloromethane, isopropyl alcohol, ethanol, methanol, acetonitrile, dimethyl sulfoxide, dimethylformamide, ethyl acetate, acetone or mixtures.

According to one embodiment of the present invention, solvents is water or dichloromethane or isopropyl alcohol or mixtures thereof.

According to one embodiment of the present invention, two different solvents are used. One of the solvents is water and isopropyl alcohol mixtures (1:1), another solvent is dichloromethane.

In one embodiment, apixaban is an active substance with dissolution problem. So, the solvents chosen should be compatible with each other and apixaban and other excipients should be easily dissolved in these solvents.

According to one embodiment of the present invention, DCM is important solvent for apixaban. But, it does not normally mix with water, so by using a co-solvent (selected from solvents group), water and DCM can be mixed. Isopropyl alcohol is a co-solvent that allows DCM and water to mix without phase separation.

According to one embodiment of the present invention, the amount of DCM is between 1.0% and 10.0%, preferably between 1.0% and 5.0% by weight in the formulation. It helps to achieve the desired dissolution at a low amount of DCM. But, in the later steps of the process, DCM is removed.

According to one embodiment of the present invention, the amount of water and isopropyl alcohol mixtures (1:1) is between 30.0% and 50.0%, preferably between 35.0% and 45.0% by weight in the formulation. But, in the later steps of the process, water and isopropyl alcohol mixtures (1:1) is removed.

Suitable binders are selected from the group comprising polyvinylpyrrolidone, sodium carboxymethyl cellulose, crospovidone, polyethylene glycol, polyvinyl alcohol, pregelatinized starch, glucose, natural gums, sucrose, sodium alginate, hydroxypropyl methyl cellulose, hydroxypropyl cellulose, carboxy methyl cellulose, methyl cellulose, gelatin, carrageenan, guar gum, carbomer, polymethacrylates, methacrylate polymers, gelatin, alginate, alginic acid, xanthan gum, hyaluronic acid, pectin, polysaccharides, carbomer, poloxamer, polyacrylamide, polyoxyethylene-alkyl ether, polydextrose, polyethylene oxide or mixtures thereof.

According to one embodiment of the present invention, the amount of binders is between 0.5% and 8.0% by weight in the total formulation. Preferably, it is between 1.0% and 6.0% or between 2.0% and 4.0% by weight in the total formulation.

According to one embodiment of the present invention, the binder is polyvinylpyrrolidone.

According to one embodiment of the invention, a tablet further comprises at least one pharmaceutically acceptable excipient which is selected from the group comprising fillers, disintegrants, surfactants, lubricants/glidants, coating agents or mixtures.

Suitable fillers are selected from the group comprising microcrystalline cellulose, lactose, mannitol, spray-dried mannitol, lactose monohydrate, dextrose, sucrose, fructose, maltose, sorbitol, xylitol, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

According to one embodiment of the present invention, the amount of fillers is between 20.0% and 95.0% by weight in the total formulation. Preferably, it is between 35.0% and 90.0% or between 50.0% and 90.0% by weight in the total formulation.

According to one embodiment of the present invention, the filler is microcrystalline cellulose or lactose or mixtures thereof.

Suitable disintegrants are selected from the group comprising crospovidone, croscarmellose sodium, starch, low-substituted hydroxypropyl cellulose, sodium carboxymethyl cellulose, calcium carboxymethyl cellulose, carboxymethyl cellulose, docusate sodium, guar gum, low substituted hydroxypropyl cellulose, polyacryline potassium, sodium alginate, corn starch, sodium starch glycolate, alginic acid, alginates, ion-exchange resins, magnesium aluminium silica, sodium dodesyl sulphate, poloxamer, sodium glycine carbonate or mixtures thereof.

According to one embodiment of the present invention, the amount of disintegrants is between 1.0% and 10.0% by weight in the total formulation. Preferably, it is between 2.0% and 8.0%, between 3.0% and 6.0% by weight in the total formulation.

According to one embodiment of the present invention, the disintegrant is crospovidone.

Suitable surfactants are selected from the group comprising sodium lauryl sulphate, sodium docusate, glyceryl monooleate, polyethylene alkyl ether, polyoxyethylene sorbitan fatty acid ester, sorbic acid, sorbitan fatty acid ester, polyoxyethylene stearates, polyethylene glycol, sodium benzoate, docusate sodium, alpha tocopherol, ascorbyl palmitate, citric acid, polyethoxylated fatty acid esters, polyoxyethylene hydrogenated castor oil or mixtures thereof.

According to one embodiment of the present invention, the amount of surfactants is between 0.5% and 5.0% by weight in the total formulation. Preferably, it is between 1.0% and 4.0% or between 1.5% and 3.5% by weight in the total formulation.

According to one embodiment of the present invention, the surfactant is sodium lauryl sulphate. Sodium lauryl sulphate is dissolved in water without precipitation when low amount of DCM (dichloromethane) is used. So, it helps to achieve the desired solution profile.

According to one embodiment of the present invention, , the weight ratio of sodium lauryl sulphate to apixaban is between 1:1 and 5:1, preferably between 1:1 and 3:1, preferably between 1:1 and 2:1. It has been observed that, this ratio is an important factor for achieving desired dissolution profile.

Suitable lubricants/glidants are selected from the group comprising from sodium stearyl fumarate, magnesium stearate, talc, colloidal silicon dioxide, corn, calcium stearate, zinc stearate, waxes, boric acid, hydrogenated vegetable oil, sodium chlorate, magnesium lauryl sulfate, sodium oleate, sodium acetate, sodium benzoate, stearic acid, fatty acid, fumaric acid, glyceryl palmito sulphate, or mixtures thereof.

According to one embodiment of the present invention, the amount of lubricants/glidants is between 0.05% and 4.0% by weight in the total formulation. Preferably, it is between 0.1% and 3.0% by weight in the total formulation.

According to one embodiment of the present invention, the lubricant/glidant is sodium stearyl fumarate.

As used here in, 'particle size distribution' means the cumulative volume size distribution as tested by any conventionally accepted method such as the laser diffraction method (i.e. malvern analysis) .The term d(0.9) means, the size at which 90% by volume of the particles are finer.

According to one embodiment of the present invention, the film coated tablet comprises apixaban having a particle size of d(0.9) greater than 100 µm.

According to one embodiment of the present invention, preferably, the film coated tablet comprises apixaban having a particle size of d(0.9) 100 µm to 150 µm.

According to one embodiment of the present invention, the tablet comprises;
- 1.0 - 10.0% by weight of apixaban
- 0.5 - 5.0% by weight of sodium lauryl sulfate
- 0.5 - 8.0% by weight of polyvinylpyrrolidone
- 1.0 - 75.0% by weight of water: isopropyl alcohol 1/1
- 1.0 - 15.0% by weight of dichloromethane
- 30.0 - 70.0% by weight of lactose
- 10.0 - 30.0% by weight of microcrystalline cellulose
- 1.0 - 10.0% by weight of crospovidone
- 0.05 - 4.0% by weight of sodium stearyl fumarate of the total tablet.

According to one embodiment of the present invention, the film coated tablet is obtained by using a wet granulation method therefore, a simple and low-cost production method was employed.

According to one embodiment of the present invention, a process for the preparation of the film coated tablet comprising apixaban comprises the following steps:
a) Dissolving at least binder in a solvent comprising water in homogenizer,
b) Adding another solvent to step (a) and mixing to obtained a solution,
c) Adding apixaban into the prepared solution at step (b) and mixing and then preparing
a granulation suspension in homogenizer.

It has surprisingly been found that a film coated tablet of excellent content uniformity, good flowability and showing improved dissolution can be obtained when at least one binder and a solvent comprising water is formulated together in wet granulation process and then adding apixaban into solvent. Wet granulation process efficiently counteracts the segregation of active agent, so it is observed that the desired stability, tablet hardness, content uniformity and compressibility.

So, an easy method is created to eliminate the disadvantages of active ingredient and also the method is simple and cost-effective method. Also, this process helps to provide the desired dissolution profile.

According to one embodiment of the present invention, the process for the preparation of the film coated tablet comprising;
a) Dissolving polyvinylpyrrolidone and sodium lauryl sulphate in water:isopropyl alcohol (1:1) in homogenizer,
b) Adding dichloromethane to step (a) and mixing to obtained a solution,
c) Adding apixaban into the prepared solution at step (b) and mixing and then preparing a granulation suspension in homogenizer.

In the process, we found that when the homogenizer is used together with DCM, it creates a synergistic effect in the tablet formulation and it helps us to use lower amounts of DCM by increasing the effectiveness of DCM. Increasing the amount of DCM adds to the dissolution problem negatively.

According to one embodiment of the present invention, water is the solvent that dissolves surfactant (SLS), binder (PVP) can dissolve in water/isopropyl alcohol/DCM.

According to one embodiment of the present invention, it is very important to use water:isopropyl alcohol (1:1) firstly as a solvent and then to use dichloromethane. This helps to provide the desired dissolution profile. The last addition of apixaban to these solvents is again important for the desired dissolution profile.

In the process, when a solution comprising water is prepared with at least binder, then mixing with the mixture comprising active agent, it was observed that the desired content uniformity is provided. Especially, the problem caused by the use of small amounts of apixaban has been prevented.

According to one embodiment of the present invention, the process for the preparation of the film coated tablet further comprises the following steps:
d) Mixing lactose, microcrystalline cellulose and crospovidone,
e) Adding the prepared granulation suspension at step (c) into powder mixture at step (d) and mixing to obtained wet granules,
f) Sieving the wet granules and then drying at fluid bed and obtained dry granules,
g) Sieving the dry granules,
h) Adding sodium stearyl fumarate and mixing,
i) Compressing to form of tablets,
j) Coating tablets with coating agents.

In the later steps of the process, the solvents are removed.

It was recognized that because of the targeted low dose for apixaban, content uniformity of the final product becomes an important product attribute. Content uniformity is a pharmaceutical analysis parameter for the quality control of tablets. Thanks to the described methods at the present invention, the tablet which have low dose of apixaban, have been developed with a desired uniformity of content.

### Example 1: The film coating tablet formulation comprising apixaban

| **Ingredients** | **% by weight** |
|---|---|
| Apixaban | 2.5 |
| Sodium lauryl sulfate | 2.5 |
| Polyvinylpyrrolidone | 3 |
| *Water : isopropyl alcohol 1/1 (v:v) | q.s. |
| *Dichloromethane (v) | q.s. |
| Lactose | 66 |
| Microcrystalline cellulose | 20.0 |
| Crospovidone | 4.0 |
| Sodium stearyl fumarate | 2.0 |
| Coating | 5 |
| **TOTAL** | **100** |

| | |
|---|---|
| * It is used in the production process. It is evaporated from the system and is not found in the finished product. q.s.= quantity sufficient | |

### Example 2: The film coating tablet formulation comprising apixaban

| **Ingredients** | **% by weight** |
|---|---|
| Apixaban | 1.0 - 10.0 |
| Sodium lauryl sulfate | 0.5 - 5.0 |
| Polyvinylpyrrolidone | 0.5 - 8.0 |
| *Water : isopropyl alcohol 1/1 (v:v) | q.s. |
| *Dichloromethane | q.s. |
| Lactose | 30.0 - 70.0 |
| Microcrystalline cellulose | 10.0 - 30.0 |
| Crospovidone | 1.0 - 10.0 |
| Sodium stearyl fumarate | 0.05 - 4.0 |
| Coating | 2.0 - 7.0 |
| **TOTAL** | **100** |

| | |
|---|---|
| * It is used in the production process. It is evaporated from the system and is not found in the finished product. q.s.= quantity sufficient | |

### Process or example 1 or 2;

a) Sodium lauryl sulfate and Polyvinylpyrrolidone K30 are dissolved with solution Agent - 1 (water: isopropyl alcohol / 1: 1) (h: h) in 'homogenizer. Solution Agent - 2 (Dichloromethane) is added to the solution and mixing is continued.
b) Apixaban is added into the prepared solution and its granulation / suspension is prepared by homogenizing with a homogenizer at ∼ 12000 rpm for 10 minutes.
c) Lactose, Microcrystalline cellulose and crospovidone are weighed and mixed.
d) The granulation agent is slowly added to the prepared powder mixture and wet granulation process is performed in the high shear mixer.
e) The wet granules obtained are sieved through a 2.8 - 4.0 mm sieve, transferred to the Fluid bed equipment for drying and dried at 60 ° C for ∼ 90 min.
f) Dry granules are sieved through a 500 µm sieve.
g) Adding sodium stearyl fumarate and mixed for a short time.
h) Compressing to form of tablets,
i) Coating tablets with coating agents.

### Preparation of the coating solution:

A sufficient amount of coating material is weighed and added at a level of 15% solids to deionized water and mixed for 45 minutes.

### Coating

| | |
|---|---|
| Hypromellose | 39.0 |
| Lactose Monohydrate | 28.0 |
| Titanium dioxide | 24.4 |
| Triacetin | 8.0 |
| Iron oxide | 0.6 |

## Claims

1. A film coated tablet comprising apixaban, at least one binder wherein at least one binder is dissolved in solvent comprising water.

2. The film coated tablet according to claim 1, wherein solvent is selected from the group comprising water, dichloromethane, isopropyl alcohol, ethanol, methanol, acetonitrile, dimethyl sulfoxide, dimethylformamide, ethyl acetate, acetone or mixtures.

3. The film coated tablet according to claim 2, wherein solvents is water or dichloromethane or isopropyl alcohol or mixtures thereof.

4. The film coated tablet according to claim 3, wherein two different solvents are used, one of the solvents is water and isopropyl alcohol mixtures (1:1), another solvent is dichloromethane.

5. The film coated tablet according to claim 1, wherein binders are selected from the group comprising polyvinylpyrrolidone, sodium carboxymethyl cellulose, crospovidone, polyethylene glycol, polyvinyl alcohol, pregelatinized starch, glucose, natural gums, sucrose, sodium alginate, hydroxypropyl methyl cellulose, hydroxypropyl cellulose, carboxy methyl cellulose, methyl cellulose, gelatin, carrageenan, guar gum, carbomer, polymethacrylates, methacrylate polymers, gelatin, alginate, alginic acid, xanthan gum, hyaluronic acid, pectin, polysaccharides, carbomer, poloxamer, polyacrylamide, polyoxyethylene-alkyl ether, polydextrose, polyethylene oxide or mixtures thereof.

6. The film coated tablet according to claim 1, further comprising at least one pharmaceutically acceptable excipient which is selected from the group comprising fillers, disintegrants, surfactants, lubricants/glidants, coating agents or mixtures.

7. The film coated tablet according to claim 6, wherein fillers are selected from the group comprising microcrystalline cellulose, lactose, mannitol, spray-dried mannitol, lactose monohydrate, dextrose, sucrose, fructose, maltose, sorbitol, xylitol, inorganic salts, calcium salts, polysaccharides, dicalcium phosphate, sodium chloride, dextrates, lactitol, maltodextrin, sucrose-maltodextrin mixture, trehalose, sodium carbonate, sodium bicarbonate, calcium carbonate or mixtures thereof.

8. The film coated tablet according to claim 6, wherein disintegrants are selected from the group comprising crospovidone, croscarmellose sodium, starch, low-substituted hydroxypropyl cellulose, sodium carboxymethyl cellulose, calcium carboxymethyl cellulose, carboxymethyl cellulose, docusate sodium, guar gum, low substituted hydroxypropyl cellulose, polyacryline potassium, sodium alginate, corn starch, sodium starch glycolate, alginic acid, alginates, ion-exchange resins, magnesium aluminium silica, sodium dodesyl sulphate, poloxamer, sodium glycine carbonate or mixtures thereof.

9. The film coated tablet according to claim 6, wherein surfactants are selected from the group comprising sodium lauryl sulphate, sodium docusate, glyceryl monooleate, polyethylene alkyl ether, polyoxyethylene sorbitan fatty acid ester, sorbic acid, sorbitan fatty acid ester, polyoxyethylene stearates, polyethylene glycol, sodium benzoate, docusate sodium, alpha tocopherol, ascorbyl palmitate, citric acid, polyethoxylated fatty acid esters, polyoxyethylene hydrogenated castor oil or mixtures thereof.

10. The film coated tablet according to claim 9, wherein surfactant is sodium lauryl sulphate.

11. The film coated tablet according to claim 1, wherein apixaban having a particle size of d(0.9) greater than 100 µm.

12. The film coated tablet according to claim 7, comprising;
a) 1.0 - 10.0% by weight of apixaban
b) 0.5 - 5.0% by weight of sodium lauryl sulfate
c) 0.5 - 8.0% by weight of polyvinylpyrrolidone
d) 1.0 - 75.0% by weight of water:isopropyl alcohol (1:1)
e) 1.0 - 15.0% by weight of dichloromethane
f) 30.0 - 70.0% by weight of lactose
g) 10.0 - 30.0% by weight of microcrystalline cellulose
h) 1.0 - 10.0% by weight of crospovidone
i) 0.05 - 4.0% by weight of sodium stearyl fumarate of the total tablet.

13. A process for the preparation of the film coated tablet comprising apixaban comprising the following steps:
a) Dissolving at least binder and at least one surfactant in a solvent comprising water in homogenizer,
b) Adding another solvent to step (a) and mixing to obtained a solution,
c) Adding apixaban into the prepared solution at step (b) and mixing and then preparing a granulation suspension in homogenizer.

14. The process for the preparation of the film coated tablet according to claim 13, comprising;
a) Dissolving polyvinylpyrrolidone and sodium lauryl sulphate in water:isopropyl alcohol (1:1) in homogenizer,
b) Adding dichloromethane to step (a) and mixing to obtained a solution,
c) Adding apixaban into the prepared solution at step (b) and mixing and then preparing a granulation suspension in homogenizer.

15. The process for the preparation of the film coated tablet according to claim 14, further comprising;
d) Mixing lactose, microcrystalline cellulose and crospovidone,
e) Adding the prepared granulation suspension at step (c) into powder mixture at step (d) and mixing to obtained wet granules,
f) Sieving the wet granules and then drying at fluid bed and obtained dry granules,
g) Sieving the dry granules,
h) Adding sodium stearyl fumarate and mixing,
i) Compressing to form of tablets,
j) Coating tablets with coating agents.
